# EUROPEAN PATENT SPECIFICATION

(11) **EP 2 632 478 B1**
(45) Date of publication and mention of the grant of the patent: **24.07.2019**
(21) Application number: 11776766.5
(22) Date of filing: 27.10.2011
(51) Int. Cl.: A61K 38/28

(54) **TREATING DIABETES MELITUS USING INSULIN INJECTIONS ADMINISTERED WITH VARYING INJECTION INTERVALS**
BEHANDLUNG VON DIABETES MELLITUS MIT IN VARIIERENDEN INJEKTIONSINTERVALLEN VERABREICHTEN INSULININJEKTIONEN
TRAITEMENT DU DIABÈTE SUCRÉ PAR INJECTIONS D'INSULINE ADMINISTRÉES À DIFFÉRENTS INTERVALLES D'INJECTION

(30) Priority: 20.06.2011 US 201161498645 P; 27.10.2010 EP 11170366; 27.10.2010 US 407206 P; 27.10.2010 EP 10189115
(43) Date of publication of application: 04.09.2013
(73) Proprietor: Novo Nordisk A/S, 2880 Bagsværd (DK)
(72) Inventor: JOHANSEN, Thue, DK-2880 Bagsvaerd (DK)
(86) International application number: PCT/EP2011/068870
(87) International publication number: WO 2012/055967

(56) References cited:
- WO-A1-2010/049488

## Description

### FIELD OF THE INVENTION

The present invention relates to a novel insulin administration scheme, which is *i.a.* useful in treatment of diabetes mellitus and hyperglycaemia, in particular of insulin-dependent diabetes mellitus. The administration of insulin and insulin involves use of analogues having a prolonged profile of action in a novel dosage regimen.

### BACKGROUND OF THE INVENTION

Diabetes mellitus often requires insulin treatment to establish proper metabolic control (comprising mainly glycaemic control, but also other metabolic parameters benefit from insulin treatment). The established practise of insulin treatment is to administer the insulin product once or more often per day, optionally in combination with other treatment modalities, as described in available treatment guidelines. Intravenous and subcutaneous insulin infusion is also used in clinical practise.

One widely used insulin treatment option is to administer a long acting insulin product, also referred to as basal insulin, to cover the insulin need of the patient wholly or partially. The long acting insulin is administered once or more often per day, at the same time every day, and is used on both type 1 diabetes and type 2 diabetes as well as for other forms of insulin requiring disease states (hyperglycaemia of any cause).

Currently, the treatment of diabetes, both type 1 diabetes and type 2 diabetes, relies to an increasing extent on the so-called intensive insulin treatment. According to this regimen, the patients are treated with multiple daily insulin injections comprising one or two daily injections of a long acting insulin, given at the same time every day, to cover the basal insulin requirement supplemented by bolus injections of a rapid acting insulin to cover the insulin requirement related to meals.

The current practice in management of diabetes and hyperglycaemia is set forth in e.g.:
- IDF Clinical Guidelines Task Force. Global Guideline for Type 2 Diabetes. Brussels: International Diabetes Federation, 2005, http://www.idf.org/webdata/docs/IDF%20GGT2D.pdf,
- IDF Clinical Guidelines Task Force. Guideline for Management of PostMeal Glucose. Brussels: International Diabetes Federation, 2007, http://www.idf.org/webdata/docs/Guideline PMG final.pdf,
- D. M. Nathan, J. B. Buse, M. B. Davidson, E. Ferrannini, R. R. Holman, R. Sherwin, and B. Zinman. Management of hyperglycemia in type 2 diabetes: a consensus algorithm for the initiation and adjustment of therapy: update regarding thiazolidinediones: a consensus statement from the American Diabetes Association and the European Association for the Study of Diabetes. Diabetes care 31 (1):173-175, 2008.

Reviews relating to basal insulin analogues and their characteristics and current clinical use can *i.a.* be found in:
- T. Heise and T. R. Pieber. Towards peakless, reproducible and long-acting insulins. An assessment of the basal analogues based on isoglycaemic clamp studies. Diabetes Obes Metab 9 (5):648-659, 2007, and
- A. H. Barnett. A review of basal insulins. Diabet Med 20 (11):873-885, 2003.

### DESCRIPTION OF THE DRAWINGS

Fig. 1 shows the glucose infusion rate plotted against the time since the administration of the trial drug.
Fig. 2 shows the blood glucose level plotted against the time since the administration of the trial drug.

### DESCRIPTION OF THE INVENTION

The present invention is based on the surprising finding that it is possible to treat a condition or disease where administration of insulin will be of benefit, such as diabetes or hyperglycaemia, by administration of insulin at intervals of varying length. For instance it has been verified that intervals of administration varying from as little as 8 hours and up to 40 hours using a dosage which at regular intervals would have been administered at 24 hour intervals provide for satisfactory diabetes treatment regimens. A number of advantages directly follow from such flexible treatment regimens:
Convenience is improved for patients by the possibility for flexible administration. For example patients can adapt the administration to their life style rather than being dependent on dosage at fixed time points, which can be advantageous in cases of incompliance or distraction where a dose is administered earlier or later than the intended time of injection; if the patient is, e.g., travelling, a child or a teenager, doing sports or a shift worker; or for any other reason has an irregular lifestyle or for whom irregularities in daily routines occur or cannot be avoided. Another example where flexible administration is advantageous is if the patient lives in a nursing home or if the patient is otherwise dependent on assisted administration of insulin. Improved convenience potentially improves patient compliance ultimately improving the long term outcome for the patient.

In one aspect the present invention provides a derivative of a naturally occurring insulin or of an insulin analogue for use in the treatment of type 1 diabetes or type 2 diabetes, comprising administering, to a patient in need thereof, effective dosages of the derivative, wherein said insulin derivative exhibits a prolonged profile of action and wherein said dosages are administered at intervals, wherein at least one of said intervals has a length of
i. at least 1.3 times the mean of said intervals, or
ii. no more than 0.85 times the mean of said intervals
wherein the mean of said intervals is at least 12 hours and less than 36 hours, wherein said dosage is not adjusted between administrations and wherein said derivative of said naturally occurring insulin or said insulin analogue is LysB29(Nε-hexadecandioyl-γ-Glu) des(B30) human insulin.

In one embodiment the method of the present invention enables use of flexibile intervals of administration, i.e. administration intervals of varying length, without compromising the glycaemic control or safety profile. An indication of the glycaemic control, such as the blood glucose or the level of HbA1c, may be determined as shown in Example 2, 3 or Example 4. An indication of the safety profile may be determined as shown in Example 2, 3 or Example 4.

### Treatment regimens of the invention

The invention is best used at the convenience of the patient. Therefore, administration intervals will be explored to allow for the presently disclosed dosage regimens. The final mode of use thus depends on the disposition and preference of the patient. This is due to the fact that the effect of any insulin depends on the insulin need of the individual patient and the sensitivity to the pharmacodynamic actions of insulin and lastly also to the preferences of the patient in a given situation. These conditions may change over time, both in terms of longer periods (years) and from day to day. From a pharmacological perspective optimal dosing intervals could be defined be the intervals giving rise to the least variation in the blood concentration levels of a particular insulin product thus giving rise to the optimal consistency in effect. Invariably, such an approach will point to the use of fixed intervals between doses. The theoretical basis for the current invention is that the variation introduced by changing dosing intervals during treatment is negligible compared to other factors affecting the variability in safety and efficacy.

Nevertheless, the present invention provides a number of embodiments of a general dosage regimen. Although an intended or optimal interval of administration of same length has been identified the present invention provides the possibility of using intervals of dosage administration of varying length.

Due to the flexible administration intervals between dosages will be of varying length and therefore the intervals are described herein as the arithmetic mean of the intervals. The expression "arithmetic mean" as used herein designates the sum of the aᵢ's divided by n, where n numbers are given and each number is denoted by aᵢ, where i = 1, ..., n. In one embodiment the mean of the intervals is determined over a period of at least 4 days, such as over a period of at least 1 week or over a period of at least 2 weeks. In one embodiment the mean of the intervals is determined over a period of at least 3 weeks, such as over a period of 6 weeks or over a period of 12 weeks. In one embodiment the mean of the intervals is determined over a period of at least 20 weeks, such as over a period of 26 weeks or over a period of 32 weeks.

In one embodiment no more than 4, such as no more than 3 or no more than 2, intervals which are adjacent to each other are no more than 0.9 times the mean of said intervals. In one embodiment no more than 4, such as no more than 3 or no more than 2, intervals which are adjacent to each other are at least than 1.1 times the mean of said intervals.

In one embodiment the invention relates to an insulin derivative for use according to the invention, wherein at least one of said intervals is no more than 0.80 times the mean of said intervals, such as no more than 0.75 times the mean of said intervals or such as no more than 0.70 times the mean of said intervals.In one embodiment the dosage is not adjusted between administrations. In one embodiment the dosage is substantially the same at every administration.

In one embodiment said intervals occur over a period of at least 3 weeks, such as at least 10 weeks or at least 26 weeks. In one embodiment said intervals occur over a period of 3 weeks, such as over a period of 10 weeks or over a period of 26 weeks.

In one embodiment at least one of said intervals is at least the mean of said intervals plus 1/24 times the mean of said intervals. In one embodiment at least one of said intervals is at least the mean of said intervals plus 1.5/24 times the mean of said intervals. In one embodiment at least one of said intervals is at least the mean of said intervals plus 2/24 times the mean of said intervals. In one embodiment at least one of said intervals is at least the mean of said intervals plus 2.5/24 times the mean of said intervals. In one embodiment at least one of said intervals is at least the mean of said intervals plus 3/24 times the mean of said intervals. In one embodiment at least one of said intervals is at least the mean of said intervals plus 3.5/24 times the mean of said intervals. In one embodiment at least one of said intervals is at least the mean of said intervals plus 4/24 times the mean of said intervals. In one embodiment at least one of said intervals is at least the mean of said intervals plus 5/24 times the mean of said intervals.

In one embodiment at least one of said intervals is no more than the mean of said intervals minus 1/24 times the mean of said intervals. In one embodiment at least one of said intervals is no more than the mean of said intervals minus 1.5/24 times the mean of said intervals. In one embodiment at least one of said intervals is no more than the mean of said intervals minus 2/24 times the mean of said intervals. In one embodiment at least one of said intervals is no more than the mean of said intervals minus 2.5/24 times the mean of said intervals. In one embodiment at least one of said intervals is no more than the mean of said intervals minus 3/24 times the mean of said intervals. In one embodiment at least one of said intervals is no more than the mean of said intervals minus 3.5/24 times the mean of said intervals. In one embodiment at least one of said intervals is no more than the mean of said intervals minus 4/24 times the mean of said intervals. In one embodiment at least one of said intervals is no more than the mean of said intervals minus 5/24 times the mean of said intervals.

In one embodiment at least one of said intervals is at least 1.1 times, such as at least 1.15 times, the mean of said intervals. In one embodiment at least one of said intervals is at least 1.2 times, such as at least 1.25 times, the mean of said intervals. In one embodiment at least one of said intervals is at least 1.3 times, such as at least 1.35 times, the mean of said intervals. In one embodiment at least one of said intervals is at least 1.4 times the mean of said intervals. In one embodiment at least one of said intervals is at least 1.45 times the mean of said intervals. In one embodiment at least one of said intervals is at least 1.5 times the mean of said intervals. In one embodiment at least one of said intervals is at least 1.55 times the mean of said intervals. In one embodiment at least one of said intervals is at least 1.6 times the mean of said intervals. In one embodiment at least one of said intervals is at least 1.65 times the mean of said intervals. In one embodiment at least one of said intervals is at least 1.7 times the mean of said intervals. In one embodiment at least one of said intervals is at least 1.75 times the mean of said intervals.

In one embodiment at least one of said intervals is no more than 0.95 times the mean of said intervals. In one embodiment at least one of said intervals is no more than 0.90 times the mean of said intervals. In one embodiment at least one of said intervals is no more than 0.85 times the mean of said intervals. In one embodiment at least one of said intervals is no more than 0.80 times the mean of said intervals. In one embodiment at least one of said intervals is no more than 0.75 times the mean of said intervals. In one embodiment at least one of said intervals is no more than 0.70 times the mean of said intervals. In one embodiment at least one of said intervals is no more than 0.65 times the mean of said intervals. In one embodiment at least one of said intervals is no more than 0.60 times the mean of said intervals. In one embodiment at least one of said intervals is no more than 0.55 times the mean of said intervals. In one embodiment at least one of said intervals is no more than 0.50 times the mean of said intervals. In one embodiment at least one of said intervals is no more than 0.45 times the mean of said intervals. In one embodiment at least one of said intervals is no more than 0.40 times the mean of said intervals. In one embodiment at least one of said intervals is no more than 0.35 times the mean of said intervals. In one embodiment at least one of said intervals is no more than 0.30 times the mean of said intervals.

In one embodiment the mean of said intervals is less than 30 hours, such as less than 24 hours or less than 18 hours. In one embodiment the mean of said intervals is at least 12 hours or at least 16 hours. In one embodiment the mean of said intervals is at least 20 hours, such as at least 24 hours or at least 28 hours.

In one embodiment the mean of said intervals is at least 12 hours, such as at least 16 hours or at least 20 hours. In one embodiment the mean of said intervals is at least 24 hours. In one embodiment the mean of said intervals is at least 28 hours, such as at least 32 hours.

In one embodiment at least one of said intervals is between 13 and 20 hours, such as between 14 and 20 hours or between 15 and 20 hours. In one embodiment at least one of said intervals is between 16 and 20 hours, such as between 17 and 20 hours or between 18 and 20 hours. In one embodiment at least one of said intervals is between 16 and 22 hours, such as between 16 and 20 hours or between 16 and 18 hours.

In one embodiment at least one of said intervals is between 16 and 16 hours, such as between 16 and 14 hours or between 16 and 12 hoursIn one embodiment substantially no other naturally occurring insulin, insulin analogue or derivative of naturally occurring insulin or insulin analogue is administered to said patient.

By **"insulin analogue"** as used herein is meant a polypeptide which has a molecular structure which formally can be derived from the structure of a naturally occurring insulin, for example that of human insulin, by deleting and/or exchanging at least one amino acid residue occurring in the naturally occurring insulin and/or adding at least one amino acid residue. The added and/or exchanged amino acid residues can either be codable amino acid residues or other naturally occurring residues or purely synthetic amino acid residues The insulin analogues may be such wherein position 28 of the B chain may be modified from the natural Pro residue to one of Asp, Lys, or Ile. In another embodiment Lys at position B29 is modified to Pro. In one embodiment B30 may be Lys and then B29 can be any codable amino acid except Cys, Met, Arg and Lys.

Also, Asn at position A21 may be modified to Ala, Gln, Glu, Gly, His, Ile, Leu, Met, Ser, Thr, Trp, Tyr or Val, in particular to Gly, Ala, Ser, or Thr and preferably to Gly. Furthermore, Asn at position B3 may be modified to Lys or Asp. Further examples of insulin analogues are des(B30) human insulin; des(B30) human insulin analogues; insulin analogues wherein PheB1 has been deleted; insulin analogues wherein the A-chain and/or the B-chain have an N-terminal extension and insulin analogues wherein the A-chain and/or the B-chain have a C-terminal extension. Thus one or two Arg may be added to position B1.

By "insulin derivative" as used herein is meant a naturally occurring insulin or an insulin analogue which has been chemically modified, e.g. by introducing a side chain in one or more positions of the insulin backbone or by oxidizing or reducing groups of the amino acid residues in the insulin or by converting a free carboxylic group to an ester group or acylating a free amino group or a hydroxy group.

With **"desB30 insulin", "desB30 human insulin"** is meant a natural insulin or an analogue thereof lacking the B30 amino acid residue. Similarly, **"desB29desB30 insulin"** or **"desB29desB30 human insulin"** means a natural insulin or an analogue thereof lacking the B29 and B30 amino acid residues.

With **"B1", "A1"** etc. is meant the amino acid residue at position 1 in the B-chain of insulin (counted from the N-terminal end) and the amino acid residue at position 1 in the A-chain of insulin (counted from the N-terminal end), respectively. The amino acid residue in a specific position may also be denoted as e.g. PheB1 which means that the amino acid residue at position B1 is a phenylalanine residue.

With **"insulin"** as used herein is meant human insulin, porcine insulin or bovine insulin with disulfide bridges between CysA7 and CysB7 and between CysA20 and CysB19 and an internal disulfide bridge between CysA6 and CysA11.

By **"parent insulin"** is meant a naturally occurring insulin such as human insulin or porcine insulin. Alternatively, the parent insulin can be an insulin analogue.

The term **"no blunting"** as used herein means that when formulated in one formulation both the rapid acting insulin and the acylated insulin has profile of action which is identical or substantially identical with the profile of action, when administering the rapid acting insulin and the acylated insulin in separate formulations.

The expression "a codable amino acid" or "a codable amino acid residue" is used to indicate an amino acid or amino acid residue which can be coded for by a triplet ("codon") of nucleotides.
hGlu is homoglutamic acid.
α-Asp is the L-form of -HNCH(CO-)CH₂COOH.
β-Asp is the L-form of -HNCH(COOH)CH₂CO-.
α-Glu is the L-form of -HNCH(CO-)CH₂CH₂COOH.
γ-Glu is the L-form of -HNCH(COOH)CH₂CH₂CO-.
α-hGlu is the L-form of -HNCH(CO-)CH₂CH₂CH₂COOH.
δ-hGlu is the L-form of -HNCH(COOH)CH₂CH₂CH₂CO-.
β-Ala is -NH-CH₂-CH₂-COOH.
Sar is sarcosine (N-methylglycine).

The expression "an amino acid residue having a carboxylic acid group in the side chain" designates amino acid residues like Asp, Glu and hGlu. The amino acids can be in either the L- or D-configuration. If nothing is specified it is understood that the amino acid residue is in the L configuration.

The expression **"an amino acid residue having a neutral side chain"** designates amino acid residues like Gly, Ala, Val, Leu, Ile, Phe, Pro, Ser, Thr, Cys, Met, Tyr, Asn and Gln.

When an insulin derivative is stated to be "soluble at physiological pH values" it means that the insulin derivative can be used for preparing injectable insulin compositions that are fully dissolved at physiological pH values. Such favourable solubility may either be due to the inherent properties of the insulin derivative alone or a result of a favourable interaction between the insulin derivative and one or more ingredients contained in the vehicle.

The following abbreviations have been used in the specification and examples:
- IDA:: Iminodiacetic acid,
- Sar:: Sarcosine (N-methyl-glycine),
- Su:: succinimidyl= 2,5-dioxo-pyrrolidin-1-yl.

LysB29(Nε-hexadecandioyl-γ-Glu) des(B30) human insulin may be provided in the form of essentially zinc free compounds or in the form of zinc complexes. When zinc complexes of LysB29(Nε-hexadecandioyl-γ-Glu) des(B30) human insulin are provided, two Zn²⁺ ions, three Zn²⁺ ions or four Zn²⁺ ions can be bound to each insulin hexamer. In one embodiment the insulin derivative is in the form of a zinc complex, wherein each insulin hexamer binds two zinc ions, three zinc ions, four zinc ions, five zinc ions, six zinc ions, seven zinc ions, eight zinc ions, nine zinc ions or ten zinc ions. Solutions of zinc complexes of the insulin derivatives will contain mixtures of such species.

Details pertaining to the preparation, formulation, pharmacology and other characteristics of relevance for LysB29(Nε-hexadecandioyl-γ-Glu) des(B30) human insulin are set forth in WO 2005/012347, which is hereby incorporated by reference herein.

In one embodiment substantially no other naturally occurring insulin, insulin analogue or derivative of naturally occurring insulin or insulin analogue exhibiting a prolonged profile of action is administered to said patient.

### Rapid acting insulin analogues

Embodiments of the Insulin derivative for use according to the invention, wherein administration of the insulin derivative exhibiting a prolonged profile of action is supplemented with more frequent administrations of a fast-acting naturally occurring insulin, insulin analogue or derivative and/or administration of a non-insulin anti-diabetic drug.

So, one embodiment the invention provides an Insulin derivative for use according to the invention, wherein said fast-acting naturally occuring insulin, insulin analogue or derivative and/or administration of a non-insulin anti-diabetic drug is AspB28 human insulin. (LysB29(Nε-hexadecandioyl-γ-Glu) des(B30) human insulin = insulin degludec (Example 4 in WO 2005/012347)). Hence, all specific disclosures in the present application which provide details relating to insulins useful in the presently disclosed invention relate mutatis mutandis to combination therapy involving the same compounds together with rapid acting insulin analogues. Typically, the rapid acting insulin is selected from the group consisting of Asp^{B28} human insulin; Lys^{B28}Pro^{B29} human insulin and Lys^{B3}Glu^{B29} human insulin. The combined product shows no blunting. The insulin derivative disclosed in WO2005/012347 can be formulated with rapid acting insulin analogues as described in WO2007/074133.

In one embodiment the invention provides an insulin derivative for use according to the present invention, wherein insulin derivative is formulated together with a pharmaceutically acceptable carrier and/or vehicle and/or diluent and/or excipient.

The insulin derivative according to the invention and the rapid acting insulin analogue can if necessary be mixed in a ratio from about 90 /10%; about 80/20%, about 70/30%, about 60/40%, about 50/50%, about 40/60%, about 30/60%, about 20/80% or about 10/90%.

### Other combinations

In one embodiment administration of the naturally occurring insulin, insulin analogue or derivative exhibiting a prolonged profile of action is supplemented with administration of a non-insulin anti-diabetic drug, such as metformin, sufonylurea and/or thia-zoledinedione or such as a GLP-1 agonist.

### Formulation of insulin, insulin analogues or derivatives thereof

In one embodiment the naturally occurring insulin, insulin analogue or derivative is formulated together with a pharmaceutically acceptable carrier and/or vehicle and/or diluent and/or excipient.

A pharmaceutical composition containing a naturally occurring insulin, an insulin analogue, or a derivative of a naturally occurring insulin or insulin analogue is termed "an insulin composition" herein. In order to exercise the present invention an insulin composition may be administered parenterally to patients in need of such a treatment. Parenteral administration may be performed by injection, such as subcutaneous, intramuscular or intravenous injection, by means of a syringe, optionally a pen-like syringe. In one embodiment the administration is by s.c. injection. In one embodiment the administration is by i.m. injection. In one embodiment the administration is by i.v. injection. Alternatively, parenteral administration can be performed by means of an infusion pump. Further options are to administer the insulin composition nasally or pulmonally, preferably in compositions, powders or liquids, specifically designed for the purpose.

Injectable insulin compositions can be prepared using the conventional techniques of the pharmaceutical industry which involve dissolving and mixing the ingredients as appropriate to give the desired end product. Thus, according to one procedure, a natural insulin, analogue or derivative is dissolved in an amount of water which is somewhat less than the final volume of the composition to be prepared. An isotonic agent, a preservative and a buffer is added as required and the pH value of the solution is adjusted - if necessary - using an acid, e.g. hydrochloric acid, or a base, e.g. aqueous sodium hydroxide as needed. Finally, the volume of the solution is adjusted with water to give the desired concentration of the ingredients.

The buffer is typically selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethan, bicine, tricine, malic acid, succinate, maleic acid, fumaric acid, tartaric acid, aspartic acid or mixtures thereof. Each one of these specific buffers constitutes an alternative useful in embodiments of the invention.

In a further embodiment of the invention the formulation further comprises a pharmaceutically acceptable preservative which may be selected from the group consisting of phenol, o-cresol, m-cresol, p-cresol, methyl p-hydroxybenzoate, propyl p-hydroxybenzoate, 2-phenoxyethanol, butyl p-hydroxybenzoate, 2-phenylethanol, benzyl alcohol, chlorobutanol, and thiomerosal, bronopol, benzoic acid, imidurea, chlorohexidine, sodium dehydroacetate, chlorocresol, ethyl p-hydroxybenzoate, benzethonium chloride, chlorphenesine (3p-chlorphenoxypropane-1,2-diol) or mixtures thereof. In a further embodiment of the invention the preservative is present in a concentration from 0.1 mg/ml to 20 mg/ml. In a further embodiment of the invention the preservative is present in a concentration from 0.1 mg/ml to 5 mg/ml. In a further embodiment of the invention the preservative is present in a concentration from 5 mg/ml to 10 mg/ml. In a further embodiment of the invention the preservative is present in a concentration from 10 mg/ml to 20 mg/ml. Each one of these specific preservatives constitutes an alternative embodiment of the invention. The use of a preservative in pharmaceutical compositions is well-known to the skilled person. For convenience reference is made to Remington: The Science and Practice of Pharmacy, 19th edition, 1995.

In a further embodiment of the invention the formulation further comprises an isotonic agent which may be selected from the group consisting of a salt (e.g. sodium chloride), a sugar or sugar alcohol, an amino acid (e.g. L-glycine, L-histidine, arginine, lysine, isoleucine, aspartic acid, tryptophan, threonine), an alditol (e.g. glycerol (glycerine), 1,2-propanediol (propyleneglycol), 1,3-propanediol, 1,3-butanediol) polyethyleneglycol (e.g. PEG400), or mixtures thereof. Any sugar such as mono-, di-, or polysaccharides, or water-soluble glucans, including for example fructose, glucose, mannose, sorbose, xylose, maltose, lactose, sucrose, trehalose, dextran, pullulan, dextrin, cyclodextrin, soluble starch, hydroxyethyl starch and carboxymethylcellulose-Na may be used. In one embodiment the sugar additive is sucrose. Sugar alcohol is defined as a C4-C8 hydrocarbon having at least one -OH group and includes, for example, mannitol, sorbitol, inositol, galactitol, dulcitol, xylitol, and arabitol. In one embodiment the sugar alcohol additive is mannitol. The sugars or sugar alcohols mentioned above may be used individually or in combination. There is no fixed limit to the amount used, as long as the sugar or sugar alcohol is soluble in the liquid preparation and does not adversely effect the stabilizing effects achieved using the methods of the invention. In one embodiment, the sugar or sugar alcohol concentration is between about 1 mg/ml and about 150 mg/ml. In a further embodiment of the invention the isotonic agent is present in a concentration from 1 mg/ml to 50 mg/ml. In a further embodiment of the invention the isotonic agent is present in a concentration from 1 mg/ml to 7 mg/ml. In a further embodiment of the invention the isotonic agent is present in a concentration from 8 mg/ml to 24 mg/ml. In a further embodiment of the invention the isotonic agent is present in a concentration from 25 mg/ml to 50 mg/ml. Each one of these specific isotonic agents constitutes an alternative embodiment of the invention. The use of an isotonic agent in pharmaceutical compositions is well-known to the skilled person. For convenience reference is made to Remington: The Science and Practice of Pharmacy, 19th edition, 1995.

Typical isotonic agents are sodium chloride, mannitol, dimethyl sulfone and glycerol and typical preservatives are phenol, m-cresol, methyl p-hydroxybenzoate and benzyl alcohol.

Examples of suitable buffers are sodium acetate, glycylglycine, HEPES (4-(2-hydroxyethyl)-1-piperazineethanesulfonic acid), TRIS (2-amino-2-hydroxymethyl-1,3-propandiol), and sodium phosphate.

A composition for nasal administration may, for example, be prepared as described in European Patent No. 272097 (to Novo Nordisk A/S).

Insulin compositions containing can be used in the treatment of states which are sensitive to insulin. Thus, they can be used in the treatment of type 1 diabetes, type 2 diabetes and hyperglycaemia for example as sometimes seen in seriously injured persons and persons who have undergone major surgery. The optimal dose level for any patient will depend on a variety of factors including the efficacy of the specific insulin, analogue or derivative employed, the age, body weight, physical activity, and diet of the patient, on a possible combination with other drugs, and on the severity of the state to be treated. It is recommended that the dosage regimen be determined for each individual patient by those skilled in the art in a similar way as for known insulin compositions, however taking into consideration the present teachings concerning dosage intervals.

Where expedient, the insulin compositions may be used in combination with other types of insulin, e.g. insulin analogues with a more rapid onset of action. Examples of such insulin analogues are described e.g. in the European patent applications having the publication Nos. EP 214826 (Novo Nordisk A/S), EP 375437 (Novo Nordisk A/S) and EP 383472 (Eli Lilly & Co.).

In one embodiment the composition of the invention is as defined in WO 2007/074133 or WO2008/152106.

### Use as a medicament

In one embodiment the present invention is used in the disease or condition is selected diabetes mellitus type 1 or 2 diabetes. In one embodiment the diabetes mellitus is type 2 diabetes, which fails oral anti-diabetic treatment.

In one embodiment the present invention relates to a naturally occurring insulin, an insulin analogue, or a derivative of a naturally occurring insulin or insulin analogue for use in a method as defined herein. In one embodiment the present invention relates to the use of a naturally occurring insulin, an insulin analogue, or a derivative of a naturally occurring insulin or insulin analogue in the preparation of a pharmaceutical composition for treatment of diabetes mellitus, wherein the treatment is as defined herein.

The present invention is further illustrated by the following examples.

### EXAMPLES

To investigate the clinical effect of an insulin product, a clinical trial has to be conducted under conditions representing the mode of use of the invention. Clinical trials investigating compounds for the treatment of diabetes with the purpose of obtaining approval and registration are subject to guidelines provided by regional authorities (the European guideline serves as an example: Note for Guidance on Clinical Investigations of Medicinal Products in the Treatment of diabetes Mellitus, EMEA, London, 2002).

As an example representing any insulin analogue with sufficiently long duration of action LysB29(Nε-hexadecandioyl-γ-Glu) des(B30) human insulin corresponding to N^{εB29}-(N^{α}-(HOOC(CH₂)₁₄CO)-γ-L-Glu) des(B30) human insulin (Example 4 in WO 2005/012347; in the following "LysB29(Nε-hexadecandioyl-γ-Glu) des(B30) human insulin") was investigated with respect to the clinical effect with varying injection intervals.

### EXAMPLE 1

*Steady state clamp* - *Investigating activity profile and duration of action of LysB29(Nε-hexadecandioyl-γ-Glu) des(B30) human insulin.*

### Methodology

The investigation was performed as a randomised, double-blind, single centre, two-period cross over trial to compare the activity profiles of LysB29(Nε-hexadecandioyl-γ-Glu) des(B30) human insulin and insulin glargine (IGlar) in subjects with type 1 diabetes.

Subjects were randomised to different sequences of subcutaneous (s.c.) multiple-dose once daily administration of LysB29(Nε-hexadecandioyl-γ-Glu) des(B30) human insulin and IGlar. The doses were either 0.57 U/kg or 0.85 U/kg of LysB29(Nε-hexadecandioyl-γ-Glu) des(B30) human insulin and 0.4 U/kg or 0.6 U/kg IGlar. The subjects were treated for 8 days for each dosing period. There was a washout period lasting 10-20 days between the two dosing periods.

At the last day of each dosing period subjects received a controlled intravenous infusion of glucose and human soluble insulin (Actrapid®) for 8-4 hours prior to trial drug administration in order to keep the blood glucose concentration stable at a level of 100 mg/dL (5.5 mmol/L), *i.e.* a euglycaemic clamp with a target blood glucose level of 100 mg/dL (5.5 mmol/L) was initiated. The euglycaemic clamp was terminated at 42 hours post-dosing but earlier if blood glucose levels increased to concentrations above 200 mg/dL (11.1 mmol/L) with no glucose infusion during the last 30 min.

Blood samples for measurement of serum LysB29(Nε-hexadecandioyl-γ-Glu) des(B30) human insulin/plasma IGlar, and blood glucose were drawn before dosing and for up to 146 hours after dosing.

Standard safety assessments were performed.

### Number of Subjects

21 subjects completed the trial.

### Diagnosis and Main Criteria for Inclusion

Male or female subjects with type 1 diabetes (≥ 12 months) aged 18-69 years (inclusive), with glycosylated haemoglobin (HbA_{1c}) ≤ 10% and normally treated with insulin (≤ 1.2 U/kg/day). Subjects should have been treated with insulin ≥ 12 months and have a body mass index (BMI) of 18-28 kg/m² (inclusive) and a fasting C-peptide < 0.3 nmol/L.

### Test Product, Dose and Mode of Administration

Multiple doses of 0.57 U/kg or 0.85 U/kg of LysB29(Nε-hexadecandioyl-γ-Glu) des(B30) human insulin, 600 nmol/ml, LysB29(Nε-hexadecandioyl-γ-Glu) des(B30) human insulin, delivered in 3 ml FlexPen® (100 DU/ml) cartridge using NovoFine® 30G, 8 mm needles.

### Duration of Treatment

Multiple doses of LysB29(Nε-hexadecandioyl-γ-Glu) des(B30) human insulin and IGlar were administered using during two different dosing periods lasting 8 days (optionally +1-5 days) at intervals of 10-20 days.

### Reference Therapy. Dose and Mode of Administration

Multiple doses (0.4 U/lg or 0.6 U/kg) of IGlar (Lantus®), 100 IU/mL, 600 nmol/mL delivered in 3.0 mL 3 mL Optiset® cartridges and injected s.c. in the thigh using PenFine® 31G, 8mm.

### Criteria for Evaluation - Efficacy

### Pharmacodynamics:

- Glucose infusion rate (GIR) during a euglycaemic clamp for 42 hours during the 8^{th} and last dosing day.
- Blood glucose concentrations.

### Pharmacokinetics:

- Serum LysB29(Nε-hexadecandioyl-γ-Glu) des(B30) human insulin/plasma IGlar concentrations for 144 hours following a single dose of either LysB29(Nε-hexadecandioyl-γ-Glu) des(B30) human insulin or IGlar.

### Primary Endpoint:

- AUCGIR(0-24h), the area under the curve (AUC) of the GIR curve from 0 to 24 hours

### Key Secondary Endpoints:

- Blood glucose level during euglycaemic clamp period
- Pharmacokinetics (tmax, terminal half-life)

### Demography of Trial Population

The 35 male and 7 female subjects with type 1 diabetes were aged 40 years on average, respectively, mean weight was 75 kg, mean HbA1c was 7.8 %, and they had a mean diabetes duration of 21 years.

### Key Results

- The AUCGIR(0-24h) for LysB29(Nε-hexadecandioyl-γ-Glu) des(B30) human insulin, did not capture the total insulin action, since pronounced levels of GIR were still present at he 24 hour time point. GIR levels at 24 hours were approximately 2.0 and 3.0 mg/kg/min for LysB29(Nε-hexadecandioyl-γ-Glu) des(B30) human insulin after the low or high dose, respectively. The corresponding values for insulin glargine were approximately 0.8 and 1.8 mg/kg/min.
- Mean GIRmax was higher for IGlar (5.6 and 4.2 mg/kg/min) than for LysB29(Nε-hexadecandioyl-γ-Glu) des(B30) human insulin (4.68 and 4.02 mg/kg/min, respectively), after the highest dose but GIRmax was equal after the lower doses (3.07 mg/kg/min).
- Mean GIR Time to GIRmax was longer for LysB29(Nε-hexadecandioyl-γ-Glu) des(B30) human insulin (13.2 hours and 6.1 for low and high dose respectively) than for IGlar (5.0 and 4.1 hours for low and high dose, respectively)
- Mean peak to trough ranges were less for LysB29(Nε-hexadecandioyl-γ-Glu) des(B30) human insulin than after insulin glargine. The values were 1.0 and 0.7 mg/kg/min after the low and high dose, respectively. For insulin glargine the corresponding values were 1.6 and 1.1 mg/kg/min.
- Average time to loss of glucose control was longer for LysB29(Nε-hexadecandioyl-γ-Glu) des(B30) human insulin than for glargine at both dose levels. This occurred after approximately 40 hours after the low LysB29(Nε-hexadecandioyl-γ-Glu) des(B30) human insulin dose and no significant loss of glucose control (defined as an increase of blood glucose of more than a 10 mg/dl) was seen at the 42 hours time point after the high LysB29(Nε-hexadecandioyl-γ-Glu) des(B30) human insulin dose. After insulin glargine dosing the loss of glucose control occurred after approximately 24 hours and 26 hours when administering the low and high dose, respectively.
- The mean time to the maximum concentration (Cmax) was shorter for insulin glargine than for LysB29(Nε-hexadecandioyl-γ-Glu) des(B30) human insulin. For insulin glargine the values were 7.2 and 6.4 hours whereas the values for LysB29(Nε-hexadecandioyl-γ-Glu) des(B30) human insulin were 9.2 and 10.1 hours after the middle and high dose, respectively.
- The mean terminal half-life was 25.2 hours (95% CI 23 to 28 hours) for LysB29(Nε-hexadecandioyl-γ-Glu) des(B30) human insulin and 13.9 hours (95% CI 13 to 15) hours for IGlar.

### Key Safety Results

In general, multiple-dose administration of LysB29(Nε-hexadecandioyl-γ-Glu) des(B30) human insulin and IGlar, respectively, was well tolerated in subjects with type 1 diabetes.

### Key Conclusions

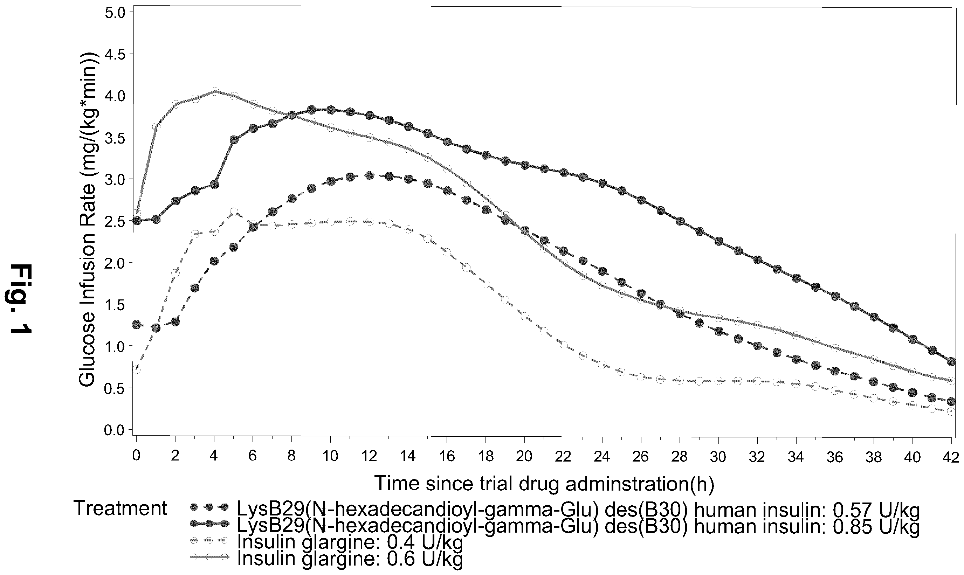

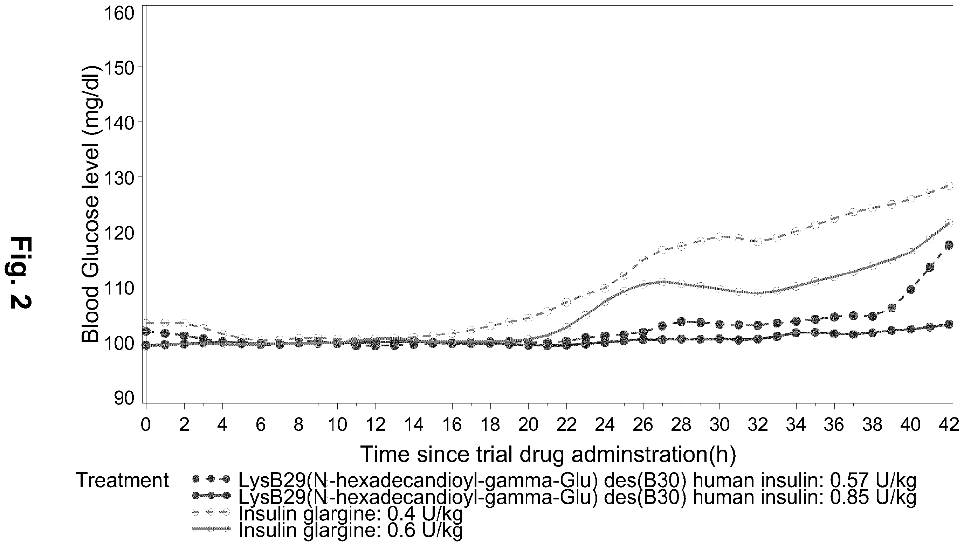

LysB29(Nε-hexadecandioyl-γ-Glu) des(B30) human insulin appeared to have a flatter and more protracted action profile and a longer duration of action compared with IGlar as evidenced by the GIR profile characteristics shown in Fig. 1. Fig. 1 shows that LysB29(Nε-hexadecandioyl-γ-Glu) des(B30) human insulin has a lower GIRmax at a comparable dose, longer time to GIRmax at both dose levels and less peak to trough range. The duration of action of LysB29(Nε-hexadecandioyl-γ-Glu) des(B30) human insulin under the present circumstances was approximately 40 hours or longer as seen in Fig. 2, which shows that LysB29(Nε-hexadecandioyl-γ-Glu) des(B30) human insulin has the ability to control blood glucose for a longer period. The conclusions based on activity data (pharmacodynamics) are supported by the pharmacokinetic data (longer time to Cmax and longer terminal half-life).

### EXAMPLE 2

*Investigating the clinical effect of* LysB29(Nε-hexadecandioyl-γ-Glu) des(B30) human insulin *administered once daily with varying intervals.*

### Key Methodological Elements and Results

The trial was designed to assess the feasibility, efficacy, safety and tolerability of LysB29(Nε-hexadecandioyl-γ-Glu) des(B30) human insulin (600 nmol/mL) for the treatment of subjects with type 2 diabetes once daily with varying injection intervals (flexible injection). The treatment consisted of administration of insulin with or without metformin and/or sufonylurea and/or pioglitazone, in subjects with type 2 diabetes failing on insulin treatment or oral antidiabetic (OAD) treatment or the combination of insulin and OAD treatment. The feasibility of varying injection intervals (i.e. flexible injection) with LysB29(Nε-hexadecandioyl-γ-Glu) des(B30) human insulin was investigated by having participating subjects inject in the morning (between waking up and breakfast) on Mondays, Wednesdays and Fridays, while injecting in the evening (between evening meal and bedtime) on Tuesdays, Thursdays, Saturdays and Sundays as shown in Table A.

**Table A. Dosing regime for flexible injection**

| | Mondays | Tuesdays | Wednesdays | Thursdays | Fridays | Saturdays | Sundays |
|---|---|---|---|---|---|---|---|
| Time of administration | Morning**¹** | Evening**²** | Morning | Evening | Morning | Evening | Evening |

| | | | | | | | |
|---|---|---|---|---|---|---|---|
| **¹**) Morning is defined as between waking up and breakfast **²**) Evening is defined as between evening meal and bedtime | | | | | | | |

### Primary Objective

To assess glucose control with respect to HbA1c after 26 weeks of treatment with LysB29(Nε-hexadecandioyl-γ-Glu) des(B30) human insulin once daily with varying injection intervals (flexible injection), LysB29(Nε-hexadecandioyl-γ-Glu) des(B30) human insulin once daily given with the evening meal or insulin glargine once daily given at the same time each day (according to the approved label), all in combination with metformin and/or sulfonylurea and/or pioglitazone in subjects with type 2 diabetes failing on insulin treatment or oral antidiabetic (OAD) treatment or the combination of insulin and OAD treatment.

### Materials and Methods

The trial was performed in subjects with type 2 diabetes, previously treated with one or more of the oral antidiabetic agents: metformin, sulfonylurea, pioglitazone or with any basal insulin treatment or the combination of the OADs specified and any basal insulin treatment. At randomisation, subjects continued their OAD treatment (if any) while adding, starting or switching to basal insulin LysB29(Nε-hexadecandioyl-γ-Glu) des(B30) human insulin once daily with varying injection intervals or LysB29(Nε-hexadecandioyl-γ-Glu) des(B30) human insulin once daily with the evening meal or insulin glargine once daily at the same time each day (according to label).

A total of 687 subjects with type 2 diabetes, age of 56 years, mean duration of diabetes of 10.6 years, mean BMI of 29.6 kg/m², mean FPG of 8.9 mmol/L, and mean HbA_{1c} of 8.4% were randomised (1:1:1) to receive once-daily LysB29(Nε-hexadecandioyl-γ-Glu) des(B30) human insulin given with varying injection intervals (229 subjects) or once-daily LysB29(Nε-hexadecandioyl-γ-Glu) des(B30) human insulin given with the evening meal (228 subjects) or once-daily insulin glargine (230 subjects), alone or in combination with metformin and/or SU and/or Pioglitazone, for a treatment period of 26 weeks.

The specific combinations of OAD and insulin treatment prior to randomisation can be seen in Table B. The insulin types used prior to randomisation to the study insulin products are shown in Table C. In table D the OAD(s) used prior to and during the experiment are shown.

**Table B Antidiabetic Treatment Regimen at Screening - Full Analysis Set**

| | IDeg OD FF | | IDeg OD | | IGlar OD | | Total | |
|---|---|---|---|---|---|---|---|---|
| | N | (%) | N | (%) | N | (%) | N | (%) |
| Number of Subjects | 229 | | 228 | | 230 | | 687 | |
| OAD only | 133 | (58.1) | 131 | (57.5) | 134 | (58.3) | 398 | (57.9) |
| One OAD | 26 | (11.4) | 44 | (19.3) | 33 | (14.3) | 103 | (15.0) |
| Two OADs | 98 | (42.8) | 79 | (34.6) | 89 | (38.7) | 266 | (38.7) |
| Three OADs | 9 | (3.9) | 8 | (3.5) | 11 | (4.8) | 28 | (4.1) |
| Four OADs | | | | | 1 | (0.4) | 1 | (0.1) |
| | | | | | | | | |
| Basal insulin only | 7 | (3.1) | 8 | (3.5) | 6 | (2.6) | 21 | (3.1) |
| Basal insulin | 7 | (3.1) | 8 | (3.5) | 6 | (2.6) | 21 | (3.1) |
| | | | | | | | | |
| Basal insulin + at least one OAD | 89 | (38.9) | 88 | (38.6) | 89 | (38.7) | 266 | (38.7) |
| Basal + one OAD | 39 | (17.0) | 31 | (13.6) | 34 | (14.8) | 104 | (15.1) |
| Basal + two OADs | 48 | (21.0) | 55 | (24.1) | 53 | (23.0) | 156 | (22.7) |
| Basal + three OADs | 2 | (0.9) | 2 | (0.9) | 2 | (0.9) | 6 | (0.9) |
| | | | | | | | | |
| Other | | | 1 | (0.4) | 1 | (0.4) | 2 | (0.3) |
| Premix + one OAD | | | 1 | (0.4) | | | 1 | (0.1) |
| Basal Bolus | | | | | 1 | (0.4) | 1 | (0.1) |

| | | | | | | | | |
|---|---|---|---|---|---|---|---|---|
| Ideg OD FF - LysB29(Nε-hexadecandioyl-γ-Glu) des(B30) human insulin administered flexibly once daily, IDeg OD - LysB29(Nε-hexadecandioyl-γ-Glu) des(B30) human insulin administered at the same time once daily, IGlar OD - Insulin glargine administered at the same time once daily N: Number of Subjects %: Proportion of Subjects | | | | | | | | |

**Table C Insulin at Screening - Summary - Full Analysis Set**

| | IDeg OD FF | | IDeg OD | | IGlar OD | | Total | |
|---|---|---|---|---|---|---|---|---|
| | N | (%) | N | (%) | N | (%) | N | (%) |
| | | | | | | | | |
| Number of Subjects | 229 | | 228 | | 230 | | 687 | |
| | | | | | | | | |
| Basal insulin | 96 | (41.9) | 96 | (42.1) | 96 | (41.7) | 288 | (41.9) |
| IDet | 19 | (8.3) | 21 | (9.2) | 21 | (9.1) | 61 | (8.9) |
| IGlar | 43 | (18.8) | 41 | (18.0) | 30 | (13.0) | 114 | (16.6) |
| NPH insulin | 34 | (14.8) | 34 | (14.9) | 45 | (19.6) | 113 | (16.4) |
| | | | | | | | | |
| Bolus insulin | | | | | 1 | (0.4) | 1 | (0.1) |
| IAsp | | | | | 1 | (0.4) | 1 | (0.1) |
| Premix | | | 1 | (0.4) | | | 1 | (0.1) |

| | | | | | | | | |
|---|---|---|---|---|---|---|---|---|
| N: Number of Subjects %: Proportion of Subjects Subjects can use more than one type of insulin within each group Insulin NPH: Neutral Protamine Hagedorn | | | | | | | | |

**Table D OAD Treatment Type at Baseline and End of Trial**

| | IDeg | | | | IGlar | | | |
|---|---|---|---|---|---|---|---|---|
| | Baseline | | End of Trial | | Baseline | | End of Trial | |
| Trial (wks) | N | (%) | N | (%) | N | (%) | N | (%) |
| 3668 (26) FF | | | | | | | | |
| | | | | | | | | |
| Biguanide | 207 | (90.4) | 206 | (90.0) | 211 | (91.7) | 212 | (92.2) |
| Metformin | 207 | (90.4) | 206 | (90.0) | 211 | (91.7) | 212 | (92.2) |
| | | | | | | | | |
| DPP-4 inhibitor | | | | | 1 | (0.4) | 1 | (0.4) |
| Sitagliptin | | | | | 1 | (0.4) | 1 | (0.4) |
| | | | | | | | | |
| Glinide | 10 | (4.4) | 10 | (4.4) | 8 | (3.5) | 7 | (3.0) |
| Repaglinide | 10 | (4.4) | 10 | (4.4) | 8 | (3.5) | 7 | (3.0) |
| | | | | | | | | |
| Sulphonylurea | 159 | (69.4) | 156 | (68.1) | 157 | (68.3) | 155 | (67.4) |
| Glibenclamide | 55 | (24.0) | 54 | (23.6) | 57 | (24.8) | 55 | (23.9) |
| Gliclazide | 43 | (18.8) | 41 | (17.9) | 39 | (17.0) | 39 | (17.0) |
| Glimepiride | 56 | (24.5) | 56 | (24.5) | 54 | (23.5) | 54 | (23.5) |
| Glipizide | 5 | (2.2) | 5 | (2.2) | 7 | (3.0) | 7 | (3.0) |
| | | | | | | | | |
| Thiazolidinedione | 13 | (5.7) | 12 | (5.2) | 17 | (7.4) | 16 | (7.0) |
| Pioglitazone | 13 | (5.7) | 12 | (5.2) | 16 | (7.0) | 15 | (6.5) |
| Rosiglitazone | | | | | 1 | (0.4) | 1 | (0.4) |
| | | | | | | | | |

| 3668 (26) | | | | | | | | |
|---|---|---|---|---|---|---|---|---|
| | | | | | | | | |
| Biguanide | 205 | (89.9) | 205 | (89.9) | | | | |
| Metformin | 205 | (89.9) | 205 | (89.9) | | | | |
| | | | | | | | | |
| Glinide | 14 | (6.1) | 13 | (5.7) | | | | |
| Repaglinide | 14 | (6.1) | 13 | (5.7) | | | | |
| | | | | | | | | |
| Sulphonylurea | 136 | (59.6) | 136 | (59.6) | | | | |
| Glibenclamide | 51 | (22.4) | 50 | (21.9) | | | | |
| Gliclazide | 37 | (16.2) | 37 | (16.2) | | | | |
| Glimepiride | 44 | (19.3) | 44 | (19.3) | | | | |
| Glipizide | 4 | (1.8) | 4 | (1.8) | | | | |
| Glyburide | | | 1 | (0.4) | | | | |
| | | | | | | | | |
| Thiazolidinedione | 17 | (7.5) | 17 | (7.5) | | | | |
| Pioglitazone | 17 | (7.5) | 17 | (7.5) | | | | |

| | | | | | | | | |
|---|---|---|---|---|---|---|---|---|
| Ideg - LysB29(Nε-hexadecandioyl-γ-Glu) des(B30) human insulin IGlar - insulin glargine, FF: Fixed injection End of Trial: a subject's last trial visit excluding the follow-up visit IGlar (3579, 3672, 3586, 3668) and Sita (3580) A subject can be on more than one OAD | | | | | | | | |

### Efficacy Results

### HbA_{1c}

HbA1c at end of trial and change in HbA1c from baseline to end of trial are given in table 1.

The confidence interval of the treatment contrast when comparing LysB29(Nε-hexadecandioyl-γ-Glu) des(B30) human insulin given with varying injection intervals with the other treatment groups was within the non-inferiority limit 0.4), which is within the non-inferiority limit accepted by the FDA (Guidance for Industry Diabetes Mellitus: Developing Drugs and Therapeutic Biologics for Treatment and Prevention, draft Guidance, U.S. Department of Health and Human Services Food and Drug Administration Center for Drug Evaluation and Research (CDER) February 2008) Thus, the group receiving LysB29(Nε-hexadecandioyl-γ-Glu) des(B30) human insulin given with varying injection intervals was similar to the other two treatment groups with respect to mean changes in HbA_{1c} from baseline to end of treatment (Table 1 and Table 2).

**Table 1. Mean HbA_{1c} after 26 Weeks of Treatment**

| | LysB29(Nε-hexadecandioyl-γ-Glu) des(B30) human insulin | LysB29(Nε-hexadecandioyl-γ-Glu) des(B30) human insulin | Insulin glargine Once daily Same time each day |
|---|---|---|---|
| | Once Daily | Once daily | |
| | Flexible Injection**²** | with the evening meal | |
| HbA_{1c} (%) after 26 weeks of treatment**¹** | 7.2 | 7.3 | 7.1 |
| Mean Change from Baseline (% points)**¹** | -1.28 | -1.07 | -1.26 |

| | | | |
|---|---|---|---|
| **¹**Arithmetic means. **²**) Flexible injection is as defined in Table A. | | | |

**Table 2. ANOVA¹ of HbA_{1c} after 26 Weeks of Treatment**

| | LysB29(Nε-hexadecandioyl-γ-Glu) des(B30) human insulin |
|---|---|
| | Once Daily |
| | Flexible Injection**²** |
| Treatment Difference vs. Insulin Glargine | 0.04 [-0.12; 0.20] |
| Once daily | |
| Same time each day (HbA1c % points [95% confidence interval]) | |
| Treatment Difference vs. LysB29(Nε-hexadecandioyl-γ-Glu) des(B30) human insulin | -0.13 [-0.29 ; 0.03] |
| Once daily | |
| with the evening meal (HbA1c % points [95% confidence interval]) | |

| | |
|---|---|
| **¹**) Results from ANOVA with treatment, anti-diabetic therapy at screening, sex, region, age and baseline HbA1c as explanatory variables. **²**) Flexible injection is as defined in Table A. | |

### Hypoglycaemia

Only six severe hypoglycaemic events were reported during the trial cf. Table 3.

**Table 3. Overview of Hypoglycaemia**

| | | | | | | | | | |
|---|---|---|---|---|---|---|---|---|---|
| | | | | | | | | | |

| | LysB29(Nε-hexadecandioyl-γ-Glu) des(B30) human insulin | | | Insulin glargine | | | LysB29(Nε-hexadecandioyl-γ-Glu) des(B30) human insulin | | |
|---|---|---|---|---|---|---|---|---|---|
| | | | | Once daily | | | | | |
| | Once Daily | | | Same time each day | | | Once daily | | |
| | Flexible Injection**⁵** | | | | | | with the evening meal | | |
| Hypoglycaemic episodes**¹** | N**²** | (%)**³** | E**⁴** | N | (%) | E | N | (%) | E |
| Severe | 1 | (0.4) | 2 | 2 | (0.9) | 2 | 2 | (0.9) | 2 |
| Documented Symptomatic | 149 | (64.8) | 841 | 124 | (54.9) | 770 | 139 | (60.7) | 803 |
| Asymptomatic | 162 | (70.4) | 879 | 159 | (70.4) | 991 | 161 | (70.3) | 892 |
| Probable Symptomatic | 20 | (8.7) | 30 | 15 | (6.6) | 29 | 18 | (7.9) | 32 |
| Relative | 27 | (11.7) | 53 | 24 | (10.6) | 35 | 26 | (11.4) | 78 |
| Unclassifiable | 12 | (5.2) | 15 | 7 | (3.1) | 11 | 7 | (3.1) | 8 |

| | | | | | | | | | |
|---|---|---|---|---|---|---|---|---|---|
| **¹**) Hypoglycaemic episodes defined as: severe = hypoglycaemic episode where food, glucagon or i.v. glucose had to be administered to the subject by another person because of severe central nervous system dysfunction associated with the hypoglycaemic episode, Documented Symptomatic= non-severe episode with subjective symptoms and plasma glucose value below 3.9 mmol/L, Asymptomatic= non-severe episode and plasma glucose value below 3.9 mmol/L and no symptoms, Probable Symptomatic= non-severe episode with no plasma glucose value but with subjective symptoms, Relative= non-severe episode with subjective symptoms and a plasma glucose value above or equal to 3.9 mmol/L. **²**) N: number of subjects. **³**) %: percentage of subjects. **⁴**) E: number of events. **⁵**) Flexible injection is as defined in Table A. | | | | | | | | | |

### Insulin Dose

**Table 4. Mean¹ Daily Insulin Dose after 26 Weeks of Treatment**

| | LysB29(Nε-hexadecandioyl-γ-Glu) des(B30) human insulin | LysB29(Nε-hexadecandioyl-γ-Glu) des(B30) human insulin | Insulin glargine |
|---|---|---|---|
| | Once daily | Once daily | Once daily |
| | Flexible injection**²** | with the evening meal | Same time each day |
| Daily Dose (U/kg) | 0.55 | 0.52 | 0.52 |

| | | | |
|---|---|---|---|
| **¹**) Arithmetic mean. **²**) Flexible injection is as defined in Table A. | | | |

### Conclusions

It was surprisingly found that using LysB29(Nε-hexadecandioyl-γ-Glu) des(B30) human insulin, which has a long duration of action and a peak-less and stable activity profile, subjects with type 2 diabetes were sufficiently regulated with once daily dosing administered with varying injection intervals alone or in combination with OAD treatment.

In subjects with type 2 diabetes failing treatment with OAD and/or insulin 26 weeks treatment with LysB29(Nε-hexadecandioyl-γ-Glu) des(B30) human insulin given flexibly (with varying injection intervals) with or without metformin and/or sulfonylurea and/or pioglitazone, resulted in comparable (non-inferior) glycaemic control and comparable incidence of hypoglycaemic episodes to that observed for LysB29(Nε-hexadecandioyl-γ-Glu) des(B30) human insulin given with the evening meal and to the glycaemic control and the incidence of hypoglycaemic episodes observed for insulin glargine given once daily at the same time each day (according to the approved label).

### EXAMPLE 3

*Investigating the clinical effect of* LysB29(Nε-hexadecandioyl-γ-Glu) des(B30) human insulin *administered once daily with varying intervals.*

### Key Methodological Elements and Results

The trial was designed to assess the feasibility, efficacy, safety and tolerability of LysB29(Nε-hexadecandioyl-γ-Glu) des(B30) human insulin (600 nmol/mL) for the treatment of subjects with type 1 diabetes once daily with varying injection intervals (flexible injection). The treatment consisted of administration of basal insulin (LysB29(Nε-hexadecandioyl-γ-Glu) des(B30) human insulin or insulin glargine) in combination with separate injections of bolus insulin,(AspB28 human insulin). The feasibility of varying injection intervals (i.e. flexible injection) with LysB29(Nε-hexadecandioyl-γ-Glu) des(B30) human insulin was investigated by having participating subjects inject in the morning (between waking up and breakfast) on Mondays, Wednesdays and Fridays, while injecting in the evening (between evening meal and bedtime) on Tuesdays, Thursdays, Saturdays and Sundays as shown in Table B.

**Table B. Dosing regime for flexible injection**

| | Mondays | Tuesdays | Wednesdays | Thursdays | Fridays | Saturdays | Sundays |
|---|---|---|---|---|---|---|---|
| Time of administration | Morning**¹** | Evening**²** | Morning | Evening | Morning | Evening | Evening |

| | | | | | | | |
|---|---|---|---|---|---|---|---|
| **¹**) Morning is defined as between waking up and breakfast **²**) Evening is defined as between evening meal and bedtime | | | | | | | |

### Primary Objective

To assess glucose control with respect to HbA1c after 26 weeks of treatment with LysB29(Nε-hexadecandioyl-γ-Glu) des(B30) human insulin once daily with varying injection intervals (flexible injection), LysB29(Nε-hexadecandioyl-γ-Glu) des(B30) human insulin once daily given with the evening meal or insulin glargine once daily given at the same time each day (according to the approved label), all in combination with AspB28 human insulin in subjects with type 1 diabetes.

### Materials and Methods

The trial was performed in subjects with type 1 diabetes, previously treated with insulin for at least 12 months. At randomisation, subjects switched to basal insulin LysB29(Nε-hexadecandioyl-γ-Glu) des(B30) human insulin once daily with varying injection intervals or LysB29(Nε-hexadecandioyl-γ-Glu) des(B30) human insulin once daily with the evening meal or insulin glargine once daily at the same time each day (according to label) all in combination with AspB28 human insulin.

A total of 490 subjects with type 1 diabetes, age of 56 years, mean duration of diabetes of 10.6 years, mean BMI of 29.6 kg/m², mean FPG of 8.9 mmol/L, and mean HbA_{1c} of 8.4% were randomised (1:1:1) to receive once-daily LysB29(Nε-hexadecandioyl-γ-Glu) des(B30) human insulin given with varying injection intervals (164 subjects) or once-daily LysB29(Nε-hexadecandioyl-γ-Glu) des(B30) human insulin given with the evening meal (165 subjects) or once-daily insulin glargine , given according to label, (161 subjects) for a treatment period of 26 weeks.

The specific insulin treatment prior to randomisation can be seen in Table C. The insulin types used prior to randomisation to the study insulin products are shown in Table D.

**Table C Anti-diabetic Treatment Regimen at Screening - Full Analysis Set**

| | IDeg OD FF | | IDeg OD | | IGlar OD | | Total | |
|---|---|---|---|---|---|---|---|---|
| | N | (%) | N | (%) | N | (%) | N | (%) |
| Number of Subjects | 164 | | 165 | | 164 | | 493 | |
| | | | | | | | | |
| Basal-Bolus Therapy | 163 | (99.4) | 165 | (100.0) | 164 | (100.0) | 492 | (99.8) |
| Basal TID + Bolus TID or more | 1 | (0.6) | | | | | 1 | (0.2) |
| Basal BID + Bolus TID or more | 50 | (30.5) | 48 | (29.1) | 44 | (26.8) | 142 | (28.8) |
| Basal OD + Bolus TID or more | 112 | (68.3) | 117 | (70.9) | 119 | (72.6) | 348 | (70.6) |
| Basal OD + Premix TID | | | | | 1 | (0.6) | 1 | (0.2) |
| | | | | | | | | |
| Other | 1 | (0.6) | | | | | 1 | (0.2) |
| Premix BID | 1 | (0.6) | | | | | 1 | (0.2) |

| | | | | | | | | |
|---|---|---|---|---|---|---|---|---|
| N: Number of Subjects %: Proportion of Subjects OD: Once daily FF: Flexible injection IDeg: LysB29(Nε-hexadecandioyl-γ-Glu) des(B30) human insulin TID: three times a day BID: two times a day Premix TID: Any biphasic insulin three times a day Premix BID:: Any biphasic insulin two times a day | | | | | | | | |

**Table D Insulin Type at Screening - Summary - Full Analysis Set**

| | IDeg OD FF | | IDeg OD | | IGlar OD | | Total | |
|---|---|---|---|---|---|---|---|---|
| | N | (%) | N | (%) | N | (%) | N | (%) |
| | | | | | | | | |
| Number of Subjects | 164 | | 165 | | 164 | | 493 | |
| | | | | | | | | |
| Basal | 163 | (99.4) | 165 | (100.0) | 164 | (100.0) | 492 | (99.8) |
| IGlar | 107 | (65.2) | 107 | (64.8) | 100 | (61.0) | 314 | (63.7) |
| IDet | 44 | (26.8) | 43 | (26.1) | 47 | (28.7) | 134 | (27.2) |
| Insulin NPH | 11 | (6.7) | 14 | (8.5) | 17 | (10.4) | 42 | (8.5) |
| IDet + Insulin NPH | 1 | (0.6) | | | | | 1 | (0.2) |
| HI | | | 1 | (0.6) | | | 1 | (0.2) |
| | | | | | | | | |
| Bolus | 163 | (99.4) | 165 | (100.0) | 163 | (99.4) | 491 | (99.6) |
| IAsp | 77 | (47.0) | 86 | (52.1) | 88 | (53.7) | 251 | (50.9) |
| ILis | 61 | (37.2) | 68 | (41.2) | 61 | (37.2) | 190 | (38.5) |
| HI | 14 | (8.5) | 6 | (3.6) | 7 | (4.3) | 27 | (5.5) |
| IGlu | 11 | (6.7) | 5 | (3.0) | 7 | (4.3) | 23 | (4.7) |
| | | | | | | | | |
| Premix | 1 | (0.6) | | | 1 | (0.6) | 2 | (0.4) |
| BIAsp | 1 | (0.6) | | | 1 | (0.6) | 2 | (0.4) |

| | | | | | | | | |
|---|---|---|---|---|---|---|---|---|
| N: Number of Subjects %: Proportion of Subjects Subjects can use more than one type of insulin within each group BIAsp: Biphasic Insulin Aspart, HI: Human Insulin IAsp: Insulin Aspart, IDet: Insulin Detemir, IGlar: Insulin Glargine IGlu: Insulin Glulisine, ILis: Insulin Lispro Insulin NPH: Neutral Protamine Hagedorn Premix: Any biphasic insulin | | | | | | | | |

### Efficacy Results

### HbA_{1c}

HbA1c at end of trial and change in HbA1c from baseline to end of trial are given in table 3. The confidence interval of the treatment contrast when comparing LysB29(Nε-hexadecandioyl-γ-Glu) des(B30) human insulin given with varying injection intervals with the other treatment groups was within the non-inferiority limit of 0.4 (Table 4).

**Table 3. Mean HbA_{1c} after 26 Weeks of Treatment**

| | LysB29(Nε-hexadecandioyl-γ-Glu) des(B30) human insulin | LysB29(Nε-hexadecandioyl-γ-Glu) des(B30) human insulin | Insulin glargine Once daily Same time each day |
|---|---|---|---|
| | Once Daily | Once daily | |
| | Flexible Injection**²** | with the evening meal | |
| HbA_{1c} (%) after 26 weeks of treatment**¹** | 7.31 | 7.30 | 7.14 |
| Mean Change from Baseline (% points)**¹** | -0.40 | -0.41 | -0.57 |

| | | | |
|---|---|---|---|
| **¹**Arithmetic means. **²**) Flexible injection is as defined in Table A. | | | |

**Table 4. ANOVA¹ of HbA_{1c} after 26 Weeks of Treatment**

| | LysB29(Nε-hexadecandioyl-γ-Glu) des(B30) human insulin |
|---|---|
| | Once Daily |
| | Flexible Injection**²** |
| Treatment Difference vs. Insulin Glargine | 0.17 [0.04; 0.30] |
| Once daily | |
| Same time each day (HbA1c % points [95% confidence interval]) | |
| Treatment Difference vs. LysB29(Nε-hexadecandioyl-γ-Glu) des(B30) human insulin | 0.01 [-0.13 ; 0.14] |
| Once daily | |
| with the evening meal (HbA1c % points [95% confidence interval]) | |

| | |
|---|---|
| **¹**) Results from ANOVA with treatment, anti-diabetic therapy at screening, sex, region, age and baseline HbA1c as explanatory variables. **²**) Flexible injection is as defined in Table A. | |

### Hypoglycaemia

Table 5 shows the hypoglycaemic events that were reported during the trial.

**Table 5. Overview of Hypoglycaemia**

| | LysB29(Nε-hexadecandioyl-γ-Glu) des(B30) human insulin | | | Insulin glargine | | | LysB29(Nε-hexadecandioyl-γ-Glu) des(B30) human insulin | | |
|---|---|---|---|---|---|---|---|---|---|
| | | | | Once daily | | | | | |
| | Once Daily | | | Same time each day | | | Once daily | | |
| | Flexible Injection**⁵** | | | | | | with the evening meal | | |
| Hypoglycaemic episodes**¹** | N**²** | (%)**³** | E**⁴** | N | (%) | E | N | (%) | E |
| Severe | 17 | (10.4) | 25 | 16 | (9.9) | 37 | 21 | (12.7) | 28 |
| Documented Symptomatic | 154 | (93.9) | 7471 | 153 | (95.0) | 7964 | 161 | (97.6) | 9467 |
| Asymptomatic | 134 | (81.7) | 3982 | 131 | (81.4) | 3978 | 139 | (84.2) | 3763 |
| Probable Symptomatic | 26 | (15.9) | 64 | 24 | (14.9) | 124 | 28 | (17.0) | 171 |
| Relative | 9 | (5.5) | 36 | 7 | (4.3) | 11 | 10 | (6.1) | 20 |
| Unclassifiable | 90 | (54.9) | 682 | 87 | (54.0) | 805 | 94 | (57.0) | 871 |

| | | | | | | | | | |
|---|---|---|---|---|---|---|---|---|---|
| **¹**) Hypoglycaemic episodes defined as: severe = hypoglycaemic episode where food, glucagon or i.v. glucose had to be administered to the subject by another person because of severe central nervous system dysfunction associated with the hypoglycaemic episode, Documented Symptomatic= non-severe episode with subjective symptoms and plasma glucose value below 3.9 mmol/L, Asymptomatic= non-severe episode and plasma glucose value below 3.9 mmol/L and no symptoms, Probable Symptomatic= non-severe episode with no plasma glucose value but with subjective symptoms, Relative= non-severe episode with subjective symptoms and a plasma glucose value above or equal to 3.9 mmol/L. **²**) N: number of subjects. **³**) %: percentage of subjects. **⁴**) E: number of events. **⁵**) Flexible injection is as defined in Table A. | | | | | | | | | |

### Insulin Dose

**Table 6. Mean¹ Daily Insulin Dose after 26 Weeks of Treatment**

| | LysB29(Nε-hexadecandioyl-γ-Glu) des(B30) human insulin | Insulin glargine | LysB29(Nε-hexadecandioyl-γ-Glu) des(B30) human insulin |
|---|---|---|---|
| | Once daily | Once daily | Once daily |
| | Flexible injection**²** | Same time each day | with the evening meal |
| Daily Basal Insulin Dose (U/kg) | 0.42 | 0.42 | 0.38 |
| Daily Prandial Insulin Dose (U/kg) | 0.35 | 0.42 | 0.33 |

| | | | |
|---|---|---|---|
| **¹**) Arithmetic mean. **²**) Flexible injection is as defined in Table A. | | | |

### Conclusions

It was surprisingly found that using LysB29(Nε-hexadecandioyl-γ-Glu) des(B30) human insulin, which has a long duration of action and a peak-less and stable activity profile, subjects with type 1 diabetes were sufficiently regulated with once daily dosing administered with varying injection intervals in combination with bolus insulin.

In subjects with type 1 diabetes 26 weeks treatment with LysB29(Nε-hexadecandioyl-γ-Glu) des(B30) human insulin given flexibly (with varying injection intervals) in combination with AspB28 human insulin, resulted in non-inferior glycaemic control and comparable incidence of hypoglycaemic episodes to that observed for LysB29(Nε-hexadecandioyl-γ-Glu) des(B30) human insulin given with the evening meal and to the glycaemic control and the incidence of hypoglycaemic episodes observed for insulin glargine given once daily at the same time each day (according to the approved label).

### EXAMPLE 4

*Investigating the clinical effect of the co-formulated combination product of* LysB29(Nε-hexadecandioyl-γ-Glu) des(B30) human insulin *and* AspB28 human insulin *administered in relation to meals with the option to change between meals from day to day during the treatment period.*

### Key methodological elements and results

The trial was designed to assess the feasibility, efficacy, safety and tolerability of combination product of LysB29(Nε-hexadecandioyl-γ-Glu) des(B30) human insulin and AspB28 human insulin (600 nmol/mL) for the treatment of subjects with type 1 diabetes once daily given in relation to a meal with the option to change from day to day the injection time for the combination product of LysB29(Nε-hexadecandioyl-γ-Glu) des(B30) human insulin and AspB28 human insulin to a different meal. The treatment consisted of the administration of the combination product of LysB29(Nε-hexadecandioyl-γ-Glu) des(B30) human insulin and AspB28 human insulin at one meal and Asp28 human insulin given in relation to remaining insulin-requiring meals in subjects with type 1 diabetes.

### Primary Objective

To assess glucose control with respect to HbA1c after 26 weeks of treatment with combination product of LysB29(Nε-hexadecandioyl-γ-Glu) des(B30) human insulin and AspB28 human insulin in relation to a selected meal (with the option of varying the meal from day to day) or insulin detemir once daily (with the option to optimise to twice daily in not optimally controlled), both treatment arms in combination with Asp28 human insulin with remaining insulin-requiring meals in subjects with type 1 diabetes.

### Materials and Methods

The trial was performed in subjects with type 1 diabetes having been diagnosed at least one year prior to entering the trial with an HbA_{1c} between 7 and 10%. At randomisation, subjects were allocated to either of two basal insulin products:
1. the combination of LysB29(Nε-hexadecandioyl-γ-Glu) des(B30) human insulin and AspB28 human insulin once daily with any meal (varying injection time from day to day) or
2. insulin detemir once daily (or twice daily) at the same time each day (according to label). Both treatment arms received AspB28 human insulin as meal time insulin at remaining meals.

A total of 548 subjects with type 1 diabetes, age of 41 years, mean duration of diabetes of 17 years, mean BMI of 26.4 kg/m², mean FPG of 10.5 mmol/L, and mean HbA_{1c} of 8.3% were randomised (2:1 in favour of the combination of LysB29(Nε-hexadecandioyl-γ-Glu) des(B30) human insulin and AspB28 human insulin for a treatment period of 26 weeks.

### Efficacy Results

### HbA_{1c}

The confidence interval of the treatment contrast when comparing LysB29(Nε-hexadecandioyl-γ-Glu) des(B30) human insulin and AspB28 human insulin with the other treatment group was within the non-inferiority limit of 0.4. The two groups were therefore similar with respect to mean changes in HbA_{1c} from baseline to end of treatment (statistical analysis Table 5).

**Table 5. Treatment difference between treatment groups in HbA1c (%) at end of trial**

| | LysB29(Nε-hexadecandioyl-γ-Glu) des(B30) human insulin and AspB28 human insulin |
|---|---|
| Treatment Difference vs. insulin detemir**¹** (HbA1c % points [95% confidence intervals]) | -0.05 [-0.18; 0.08] |

| | |
|---|---|
| **¹**) Results from ANOVA with treatment, anti-diabetic therapy at screening, sex, region, age and baseline HbA1c as explanatory variables | |

### Hypoglycaemia

Hypoglycaemic episodes were registered during the trial according to the definitions of American Diabetes Assocation, cf. Table 6.

**Table 6. Overview of Hypoglycaemia. Randomisation was 2:1 (LysB29(Nε-hexadecandioyl-γ-Glu) des(B30) human insulin and AspB28 human insulin : insulin detemir)**

| | LysB29(Nε-hexadecandioyl-γ-Glu) des(B30) human insulin and AspB28 human insulin | | | Insulin Detemir | | |
|---|---|---|---|---|---|---|
| Hypoglycaemic episodes**¹** | N**²** | (%)**³** | E**⁴** | N | (%) | E |
| Severe | 35 | 9.7 | 56 | 22 | 12.2 | 35 |
| Documented Symptomatic | 319 | 88.1 | 9670 | 156 | 86.7 | 5126 |
| Asymptomatic | 270 | 74.6 | 4032 | 137 | 76.1 | 1804 |
| Probable Symptomatic | 71 | 19.6 | 234 | 40 | 22.2 | 149 |
| Relative | 42 | 11.6 | 108 | 17 | 9.4 | 33 |
| Unclassifiable | 105 | 29.0 | 395 | 50 | 27.8 | 241 |

| | | | | | | |
|---|---|---|---|---|---|---|
| **¹**) Hypoglycaemic episodes defined as: severe = hypoglycaemic episode where food, glucagon or i.v. glucose had to be administered to the subject by another person because of severe central nervous system dysfunction associated with the hypoglycaemic episode, Documented Symptomatic= non-severe episode with subjective symptoms and plasma glucose value below 3.9 mmol/L, Asymptomatic= non-severe episode and plasma glucose value below 3.9 mmol/L and no symptoms, Probable Symptomatic= non-severe episode with no plasma glucose value but with subjective symptoms, Relative= non-severe episode with subjective symptoms and a plasma glucose value above or equal to 3.9 mmol/L. **²**) N: number of subjects. **³**) %: percentage of subjects. **⁴**) E: number of events. | | | | | | |

### Insulin Dose

**Table 7. Mean¹ Total Daily Insulin Dose after 26 Weeks of Treatment**

| | LysB29(Nε-hexadecandioyl-γ-Glu) des(B30) human insulin and AspB28 human insulin + AspB28 human insulin | Insulin Detemir + AspB28 human insulin |
|---|---|---|
| Daily Dose (U/kg) | 0.86 | 1.00 |

| | | |
|---|---|---|
| **¹**) Arithmetic mean. | | |

### Conclusions

It was surprisingly found that the basal component of the combination product, LysB29(Nε-hexadecandioyl-γ-Glu) des(B30) human insulin, which has a long duration of action and a peak-less and stable activity profile, enabled subjects to be sufficiently regulated with once daily dosing even when varying the injection intervals as a result of changing the meal at which injection of the combination product was administered.

In subjects with type 1 diabetes insulin 26 weeks treatment with LysB29(Nε-hexadecandioyl-γ-Glu) des(B30) human insulin combined with AspB28 human insulin given once daily in relation to a selected meal (with the option of varying the injection time to a different meal from day to day) resulted in comparable (non-inferior) glycaemic control to that of insulin detemir given twice daily according to label, both treatment were combined with AspB28 human insulin for the remaining meals. The combination of LysB29(Nε-hexadecandioyl-γ-Glu) des(B30) human insulin resulted in lower insulin use and a lower incidence of hypoglycaemic episodes compared to that observed for insulin detemir.

*The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.*

*The citation and incorporation of patent documents herein is done for convenience only and does not reflect any view of the validity, patentability, and*/*or enforceability of such patent documents.*

## Claims

1. Derivative of a naturally occurring insulin or of an insulin analogue for use in the treatment of type 1 diabetes or type 2 diabetes, comprising administering, to a patient in need thereof, effective dosages of the derivative, wherein said insulin derivative exhibits a prolonged profile of action and wherein said dosages are administered at intervals, wherein at least one of said intervals has a length of
i. at least 1.3 times the mean of said intervals, or
ii. no more than 0.85 times the mean of said intervals
wherein the mean of said intervals is at least 12 hours and less than 36 hours, wherein said dosage is not adjusted between administrations and wherein said derivative of said naturally occurring insulin or said insulin analogue is LysB29(Nε-hexadecandioyl-γ-Glu) des(B30) human insulin.

2. Insulin derivative for use according to claim 1, wherein at least one of said intervals has a length of at least 1.35 times the mean of said intervals, such as at least 1.4 times the mean of said intervals or such as at least 1.45 times the mean of said intervals.

3. Insulin derivatives for use according to any of the preceding claims, wherein at least one of said intervals is no more than 0.80 times the mean of said intervals, such as no more than 0.75 times the mean of said intervals or such as no more than 0.70 times the mean of said intervals.

4. Insulin derivative for use according to any one of the preceding claims, wherein administration of the insulin derivative exhibiting a prolonged profile of action is supplemented with more frequent administrations of a fast-acting naturally occurring insulin, insulin analogue or derivative and/or administration of a non-insulin anti-diabetic drug.

5. Insulin derivative for use according to claim 4, wherein said fast-acting naturally occuring insulin, insulin analogue or derivative and/or administration of a non-insulin anti-diabetic drug is AspB28 human insulin.

6. Insulin derivative for use according to claim 5, wherein the LysB29(Nε-hexadecandioyl-γ-Glu) des(B30) human insulin and the AspB28 human insulin is mixed in a ratio of 70/30%.

7. Insulin derivative for use according to any one of the preceding claims, wherein substantially no other naturally occurring insulin, insulin analogue or derivative of naturally occurring insulin or insulin analogue exhibiting a prolonged profile of action is administered to said patient.

8. Insulin derivative for use according to any one of the preceding claims, wherein insulin derivative is formulated together with a pharmaceutically acceptable carrier and/or vehicle and/or diluent and/or excipient.

## Patentansprüche

1. Derivat eines natürlich auftretenden Insulins oder eines Insulinanalogs zur Verwendung bei der Behandlung von Typ-I-Diabetes oder Typ-II-Diabetes, ein Verabreichen effektiver Dosierungen des Derivats an einen Patienten, der dessen bedarf, umfassend, wobei dieses Insulinderivat ein verlängertes Wirkungsprofil aufweist und wobei diese Dosierungen in Intervallen verabreicht werden, wobei zumindest eines dieser Intervalle eine Länge von
i. zumindest dem 1,3-fachen des Durchschnitts dieser Intervalle beträgt, oder eine Länge von
ii. nicht mehr als dem 0,85-fachen des Durchschnitts dieser Intervalle beträgt,
wobei der Durchschnitt dieser Intervalle zumindest 12 Stunden und weniger als 36 Stunden beträgt, wobei diese Dosis nicht zwischen den Verabreichungen angepasst wird und wobei dieses Derivat dieses natürlich auftretenden Insulins oder dieses Insulinanalogs LysB29(Nε-Hexadecandioyl-γ-Glu) des(B30)-Humaninsulin ist.

2. Insulinderivat zur Verwendung nach Anspruch 1, wobei zumindest eines dieser Intervalle eine Länge von zumindest dem 1,35-fachen des Durchschnitts dieser Intervalle beträgt, wie etwa zumindest das 1,4-fache des Durchschnitts dieser Intervalle oder wie etwa zumindest das 1,45-fache des Durchschnitts dieser Intervalle.

3. Insulinderivate zur Verwendung nach einem der vorhergehenden Ansprüche, wobei zumindest eines dieser Intervalle nicht mehr als das 0,80-fache des Durchschnitts dieser Intervalle beträgt, wie etwa nicht mehr als das 0,75-fache des Durchschnitts dieser Intervalle oder wie etwa nicht mehr als das 0,70-fache des Durchschnitts dieser Intervalle.

4. Insulinderivat zur Verwendung nach einem der vorhergehenden Ansprüche, wobei das Verabreichen des Insulinderivats, das ein verlängertes Wirkungsprofil aufweist, mit häufigeren Verabreichungen eines schnell wirkenden natürlich auftretenden Insulins, Insulinanalogs oder Derivats und/oder der Verabreichung eines antidiabetischen Medikaments, das nicht Insulin ist, ergänzt wird.

5. Insulinderivat zur Verwendung nach Anspruch 4, wobei dieses schnell wirkende natürlich auftretende Insulin, Insulinanalog oder Derivat und/oder die Verabreichung eines antidiabetischen Medikaments, das nicht Insulin ist, AspB28-Humaninsulin ist.

6. Insulinderivat zur Verwendung nach Anspruch 5, wobei das LysB29(Nε-Hexadecandioyl-γ-Glu) des(B30)-Humaninsulin und das AspB28-Humaninsulin in einem Verhältnis von 70/30 % gemischt werden.

7. Insulinderivat zur Verwendung nach einem der vorhergehenden Ansprüche, wobei dem Patienten im Wesentlichen kein weiteres natürlich auftretendes Insulin, Insulinanalog oder Derivat eines natürlich auftretenden Insulins oder Insulinanalogs, das ein verlängertes Wirkungsprofil aufweist, verabreicht wird.

8. Insulinderivat zur Verwendung nach einem der vorhergehenden Ansprüche, wobei das Insulinderivat zusammen mit einem pharmazeutisch unbedenklichen Träger und/oder Vehikel und/oder Verdünnungsmittel und/oder Hilfsstoff formuliert wird.

## Revendications

1. Dérivé d'une insuline naturelle ou d'un analogue d'insuline destiné à être utilisé dans le traitement du diabète de type 1 ou du diabète de type 2, comprenant l'administration, à un patient dans le besoin, des posologies efficaces du dérivé, dans lequel ledit dérivé d'insuline présente un profil d'action prolongé et dans lequel lesdites posologies sont administrées à intervalles, dans lequel au moins l'un des intervalles a une longueur
i. d'au moins 1,3 fois la moyenne desdits intervalles, ou
ii. d'au plus 0,85 fois la moyenne desdits intervalles
où la moyenne desdits intervalles est d'au moins 12 heures et inférieure à 36 heures, où ladite dose n'est pas ajustée entre les administrations et où ledit dérivé de ladite insuline d'origine naturelle ou ledit analogue d'insuline est l'insuline humaine des(B30) Lysb29(Nε-hexadecandioyl-γ-Glu).

2. Dérivé d'insuline destiné à être utilisé selon la revendication 1, dans lequel au moins un desdits intervalles a une longueur d'au moins 1,35 fois la moyenne desdits intervalles, par exemple au moins 1,4 fois la moyenne desdits intervalles ou par exemple au moins 1,45 fois la moyenne desdits intervalles.

3. Dérivés d'insuline destinés à être utilisés selon l'une quelconque des revendications précédentes, dans lequel au moins l'un de ces intervalles n'est pas supérieur à 0,80 fois la moyenne desdits intervalles, par exemple pas plus de 0,75 fois la moyenne desdits intervalles ou par exemple pas plus de 0,70 fois la moyenne desdits intervalles.

4. Dérivé d'insuline destiné à être utilisé selon l'une quelconque des revendications précédentes, dans lequel l'administration du dérivé d'insuline présentant un profil d'action prolongé est complétée par des administrations plus fréquentes d'une insuline d'origine naturelle à action rapide, d'un analogue d'insuline ou d'un dérivé et/ou d'une administration d'un médicament antidiabétique non insulinique.

5. Dérivé d'insuline destiné à être utilisé selon la revendication 4, dans lequel ladite insuline d'origine naturelle à action rapide, l'analogue de l'insuline ou le dérivé et/ou l'administration d'un médicament antidiabétique non insulinique est l'insuline humaine AspB28.

6. Dérivé d'insuline destiné à être utilisé selon la revendication 5, dans lequel l'insuline humaine des(B30) Lysb29(Nε-hexadecandioyl-γ-Glu) et l'insuline humaine AspB28 sont mélangées dans un rapport de 70/30 %.

7. Dérivé d'insuline destiné à être utilisé selon l'une quelconque des revendications précédentes, dans lequel, essentiellement aucune autre insuline d'origine naturelle, analogue d'insuline ou dérivé d'insuline d'origine naturelle ou d'analogue d'insuline présentant un profil d'action prolongé n'est administré audit patient.

8. Dérivé d'insuline à utiliser selon l'une quelconque des revendications précédentes, dans lequel le dérivé d'insuline est formulé avec un excipient et/ou un diluant et/ou un véhicule et/ou un support pharmaceutiquement acceptable.
